# FASCICULE DE BREVET EUROPEEN

(11) **EP 0 693 133 B1**
(45) Date de publication et mention de la délivrance du brevet: **21.01.1998**
(21) Numéro de dépôt: 94913140.3
(22) Date de dépôt: 06.04.1994
(51) Int. Cl.: C12P 23/00, C12N 1/06, C12P 1/00

(54) **PROCEDE D'EXTRACTION DE CAROTENOIDES ET NOTAMMENT D'ASTAXANTHINE A PARTIR D'UNE CULTURE DE MICRO-ALGUES**
VERFAHREN ZUR EXTRAKTION VON CAROTENOIDEN, INSBESONDERE VON ASTAXANTHIN, AUS EINER KULTUR VON MIKROALGEN
METHOD FOR EXTRACTING CAROTENOIDS, PARTICULARLY ASTAXANTHIN, FROM A CULTURE OF MICROALGAE

(30) Priorité: 07.04.1993 FR 9304119
(43) Date de publication de la demande: 24.01.1996
(73) Titulaire: THALLIA Pharmaceuticals S.A., 69130 Ecully (FR)
(72) Inventeur: GUDIN, Claude, F-13500 Martigues (FR); TREZZY, Claudine, F-13740 Lerove (FR); MAILLARD, Patrick, F-84120 Perthuis (FR)
(74) Mandataire: Pernez, Helga
(86) Numéro de dépôt international: FR9400383
(87) Numéro de publication internationale: WO9423057

(56) Documents cités:
- WO-A-89/06910
- US-A- 4 851 339
- DATABASE WPI Section Ch, Week 9317, Derwent Publications Ltd., London, GB; Class B05, AN 93-136904 & JP,A,5 068 585 (HIGASHIMARU SHOYU KK) 23 Mars 1993
- CHEMICAL ABSTRACTS, vol. 83, no. 15, 13 Octobre 1975, Columbus, Ohio, US; abstract no. 128497y, SIGALAT, C. & DE KOUCHKOVSKY, Y. 'Fractionation and characterization of the photosynthetic apparatus of the unicellular blue-green alga Anacystis nidulans. I. Preparation of membrane fractions by osmotic lysis and pigment analysis' page 249 ;

## Description

La présente invention a pour objet un procédé d'extraction de caroténoïdes et plus spécialement d'astaxanthine à partir d'une culture de micro-algues et en particulier, *d'Haematococcus pluvialis.* Elle s'applique avantageusement dans le domaine de l'agro-alimentaire et notamment en pisciculture pour la production de nourriture pour les poissons et les crustacés ainsi qu'en pharmacologie.

La coloration des micro-algues est due à des pigments qui sont à l'origine des réactions photochimiques de la photosynthèse tels que les chlorophylles, les phycobilines et les caroténoïdes. En particulier l'astaxanthine assure la pigmentation en rose de la chair des crevettes, des truites, des saumons, etc.

Les caroténoïdes contenus dans *Haematococcus pluvialis* sont le β-carotène, l'adonirubine, la lutéine, l'échinenone, l'astaxanthine, la violaxanthine, la néoxanthine, la cantaxanthine et de façon plus générale les dérivés mono-, di- et tri-esters de l'astaxanthine.

Le procédé de l'invention s'applique aussi aux micro-algues du type *Dunaliella bardawil* et *Dunaliella satina* contenant du β-carotène ; *Chlorella pyrenoïdosa* contenant de la lutéine ; *Vacularia virescens* contenant de la *diadinoxanthine.* En outre, l'astaxanthine est contenue dans *Chlamydomonas nivalis, Euglena héliorobrescens et sanguinea, Balticola droebakensis.*

L'invention ne porte que sur l'extraction des caroténoïdes d'une culture de micro-algues, la production de ces caroténoïdes étant réalisée conformément à l'art antérieur et en particulier, comme décrit dans les références FR-A-2 620 131 et EP-A-329 754

Il est connu du document WO-A-90/05765 d'extraire des caroténoïdes et notamment de l'astaxanthine à partir de déchets de plantes, d'algues ou de crustacés en utilisant une solution bouillante alcaline (>95°C). L'astaxanthine et les caroténoïdes associés sont retirés du produit extrait soit par précipitation acide, soit par refroidissement, retrait et séparation de phases puis épuration ultérieure.

Il est en outre connu d'extraire des caroténoïdes et notamment du β-carotène à partir d'une culture de *Dunaliella salina* par le document US-A-4 680 314 en ajoutant une huile végétale comestible en fin de culture de la suspension de *Dunaliella.* Cette huile attire en surface les algues huileuses riches en caroténoides que l'on récolte. Pour séparer le β-carotène des cellules des micro-algues, on modifie le pH en acidifiant le milieu.

Un autre procédé d'extraction de caroténoïdes à partir de *Dunaliella* est décrit dans le document AU-A-487 018. Ce procédé consiste à agiter fortement la culture, en présence d'un solvant organique partiellement miscible au milieu aqueux de la culture, jusqu'à éclatement des cellules de micro-algues puis dissolution des caroténoïdes dans le solvant organique.

Ce solvant contient alors des caroténoïdes et des déchets de micro-algues et est ensuite filtré sur une poudre de diatomées sous vide partiel. Le filtrat récolté contient environ 1% en poids de matière sèche de β-carotène des micro-algues. Ce β-carotène est ensuite concentré.

Ces procédés d'extraction connus présentent l'inconvénient d'un rendement faible et donc d'un prix de revient élevé.

L'invention a pour objet un nouveau procédé d'extraction des caroténoïdes à partir de micro-algues présentant un rendement supérieur d'au moins 4 fois ceux de l'art antérieur.

A cet effet, l'invention a pour objet un procédé d'extraction de caroténoïdes à partir d'une culture de micro-algues, consistant à :
**a)** - centrifuger le milieu de culture pour former une pâte riche en micro-algues ;
**b)** - diluer la pâte dans une solution saline pour provoquer un choc osmotique sur les cellules des micro-algues ;
**c)** - acidifier la solution et la faire bouillir pour ramollir la paroi des cellules ;
**d)** - filtrer la solution pour séparer les cellules acidifiées de la solution ;
**e)** - laver les cellules obtenues en d) à l'aide d'un solvant organique apte à solubiliser les caroténoïdes contenus dans les cellules.

Ce procédé d'extraction est applicable à toutes les micro-algues citées précédemment.

Le choc osmotique permet de fragiliser le réseau endoplasmique des micro-algues qui retient les globules de caroténoïdes, appelés haematochromes.

Le choc osmotique peut être réalisé avec un sel d'acide et de bases fortes comme NaCl, KCl, SO₄Na, CaCl₂, KSO₄, etc. La concentration de ce sel peut aller de 50 à 300g/l.

L'acidification est réalisée avec un acide fort minéral ou organique, de façon à obtenir un pH au plus égal à 4. En particulier, on utilise un acide minéral tel que l'acide chlorhydrique ou l'acide sulfurique.

La durée de l'ébullition est fonction de la nature des cellules et notamment de leur âge et selon si ces cellules sont enkystées ou non. En pratique, cette étape dure de 10 min à 1 h.

L'hydrolyse acide à chaud de l'invention a pour but de ramollir et de rendre plus hydrophile la paroi des cellules contenant les haematochromes et surtout les kystes contenant ces cellules.

Le solvant organique utilisé pour solubiliser les caroténoïdes sont des esters, des alcools, des cétones, ou encore des alcanes éventuellement halogénées. En particulier, on utilise une cétone telle que l'acétone, ou encore le dichlorométhane.

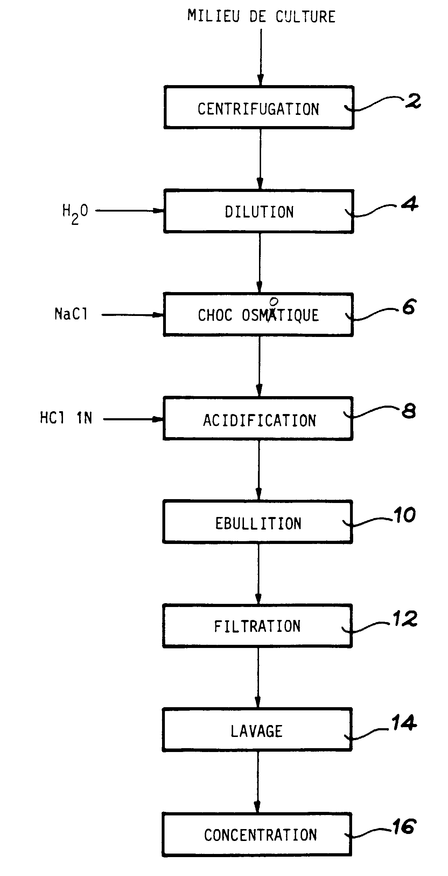

D'autres caractéristiques et avantages de l'invention ressortiront mieux de la description qui va suivre, donnée à titre illustratif et non limitatif en référence à l'unique figure annexée qui illustre les différentes étapes du procédé de l'invention.

Ce procédé est parfaitement bien adapté à l'extraction d'astaxanthine et de ses esters dérivés à partir *d'Haematococcus pluvialis.*

Aussi, la description qui suit se rapportera à l'extraction de caroténoïdes à partir d'une culture *d'Haematococcus pluvialis,* bien que l'invention s'applique à toutes les micro-algues contenant des caroténoïdes et en particulier à celles citées précédemment.

En fin de culture des micro-algues *d'Haematococcus,* par exemple selon le procédé du document EP-A-329 754, on effectue comme représenté en 2 sur la figure annexée une centrifugation pour récolter une pâte rouge, contenant les caroténoïdes totaux à une concentration de 1 à 6% du poids de matière sèche. Cette centrifugation est réalisée entre 5000g et 40000g et de préférence entre 20000g et 35000g. La pâte recueillie contient 30 à 70% en poids de caroténoïdes.

Cette pâte est ensuite diluée avec de l'eau, comme représenté en 4, de façon à arriver à une concentration de 10 à 100g/l de matière sèche de biomasse. Cette solution est alors additionnée de NaCl à raison de 200g/l pour provoquer un choc osmotique sur les cellules *d'Haematococcus* fragilisant le réseau endoplasmique de ces cellules. Cette étape porte la référence 6.

La solution est alors acidifiée, comme indiqué en 8 par addition d'HCl 1N de façon à obtenir un pH de 2. Cette solution est alors portée à ébullition pendant 10 min à 1 h selon l'âge des cellules, et leur enkystement. Cette étape porte la référence 10.

On effectue ensuite un refroidissement de la solution, puis une filtration 12 par exemple sur filtre de verre à température ambiante. On obtient ainsi d'une part un filtrat limpide et incolore et d'autre part sur le filtre la biomasse hydrolysée, bien rouge.

Le concentrat est lavé avec de l'acétone, comme indiqué en 14, qui peu à peu se décolore pour donner un filtrat rouge contenant de l'astaxanthine, ses dérivés mono-, di- et tri-esters ainsi que des traces de β-carotène, d'astaxanthine libre, d'échinenone et de cantaxanthine libre.

Dès que sorti de la cellule, cet extrait acétonique n'est plus photostable, c'est-à-dire que sa couleur est instable en lumière naturelle.

Pour concentrer l'astaxanthine et ses dérivés, on effectue ensuite une évaporation de l'acétone entre 20 et 40°C pendant 5 à 30 minutes, couduisant à un résidu huileux rouge que l'on peut alors redissoudre dans tout solvant compatible avec l'astaxanthine tel que le dichlorométhane. Cet extrait concentré en astaxanthine doit être conservé à l'obscurité, et à basse température (0 à 5°C) et éventuellement sous atmosphère d'azote car il est photodestructible.

Cet extrait contient en outre, par identification par spectromètre de masse, des résidus de phéophytine et de triglycéride colorés en rouge.

Le procédé de l'invention a été comparé à une méthode plus traditionnelle qui consistait à broyer les micro-algues lyophilisées dans de l'acétone entre 5°C et 7°C dans un broyeur rotatif tournant à 20000 t/min sous atmosphère d'argon, à l'obscurité. Le procédé de l'invention et ce procédé classique ont été mis en oeuvre sur quatre échantillons identiques. Les résultats sont portés dans le tableau ci-après.

On constate, à la lecture de ce tableau que le rendement d'extraction du procédé de l'invention est environ 4 fois supérieur à celui des méthodes classiques actuellement utilisées.

Par ailleurs, des tests d'extraction ont été réalisés sur des échantillons de kystes rouges provenant d'une efflorescence naturelle *d'Haematococcus pluvialis* avec le procédé de l'invention et celui connu sous le nom de Bligh Dyers décrit dans le document Bligh, E.G. and W.J. Dyer (1959) Can. J. Biochem. Physiol. 31, 911. Ce procédé connu utilise un mélange de chloroforme, de méthanol et d'eau pour l'extraction de caroténoïdes.

Sur un même échantillon, avec la méthode de Bligh Dyers, on obtient 0,64% de caroténoïdes totaux par rapport au poids de matière sèche au lieu de 2,68% en poids de caroténoïdes avec le procédé de l'invention.

L'originalité de l'invention est la brutalité de l'extraction (choc osmotique) sur des pigments réputés extrêmement fragiles et labiles à la photo-oxydation quand ils sont purs. Ce choc osmotique a pour but de rompre peu à peu les formes stables dans lesquelles sont engagés ces composés fragiles afin de les rendre disponibles et utilisables.

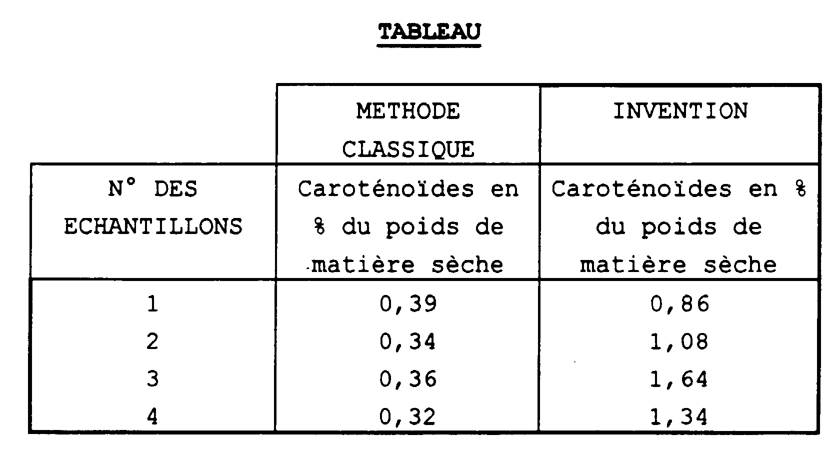

## Revendications

1. Procédé d'extraction de caroténoïdes à partir d'une culture de micro-algues, consistant à :
**a)** - centrifuger (2) le milieu de culture pour former une pâte riche en micro-algues ;
**b)** - diluer (4, 6) la pâte dans une solution saline pour provoquer un choc osmotique sur les cellules des micro-algues ;
**c)** - acidifier (8) la solution et la faire bouillir (10) pour ramollir la paroi des cellules ;
**d)** - filtrer (12) la solution pour séparer les cellules acidifiées de la solution ;
**e)** - laver (14) les cellules obtenues en d) à l'aide d'un solvant organique apte à solubiliser les caroténoïdes contenus dans les cellules.

2. Procédé selon la revendication 1, caractérisé en ce que la solution saline est une solution de NaCl.

3. Procédé selon la revendication 2, caractérisé en ce que la solution saline contient de 50 à 300 g/l de NaCl.

4. Procédé selon l'une quelconque des revendications 1 à 3, caractérisé en ce que la solution est acidifiée à un pH de 2 à 4.

5. Procédé selon l'une quelconque des revendications 1 à 4, caractérisé en ce que l'ébullition (10) est effectuée pendant 10 minutes à 1 heure.

6. Procédé selon l'une quelconque des revendications 1 à 5, caractérisé en ce que le solvant organique est de l'acétone ou du dichlorométhane.

7. Procédé selon l'une quelconque des revendications 1 à 6, caractérisé en ce qu'on effectue une étape de concentration (16) des caroténoïdes contenus dans le solvant organique.

8. Procédé d'extraction d'astaxanthine et de ses esters dérivés à partir d'une culture *d'Haemotococcus pluvialis,* caractérisé en ce qu'on utilise le procédé selon l'une quelconque des revendications 1 à 7.

## Patentansprüche

1. Extraktionsverfahren von Karotenoiden aus eine Mikroalgenkultur, das darin besteht:
a) - das Kulturmilieu zu zentrifugieren (2), um eine Paste reich an Mikroalgen zu bilden;
b) - die Paste in einer Salzlösung zu verdünnen (4, 6), um einen osmotischen Schock auf den Zellen der Mikroalgen zu verursachen;
c) - die Lösung zu säuern (8) und zu kochen (10), um die Zellenwände zu erweichen;
d) - die Lösung zu filtern (12), um die gesäuerten Zellen von der Lösung zu trennen;
e) - die unter d) erhaltenen Zellen mit einem organischen Lösemittel zu waschen (14), das geeignet ist, die in den Zellen enthaltenen Karotenoide aufzulösen.

2. Verfahren nach Anspruch 1, dadurch gekennzeichnet, dass die Salzlösung eine NaC1-Lösung ist.

3. Verfahren nach Anspruch 2, dadurch gekennzeichnet, dass die Salzlösung 50 bis 300 g/l NaCl enthält.

4. Verfahren nach einem der Ansprüche 1 bis 3, dadurch gekennzeichnet, dass die Lösung mit einem pH von 2 bis 4 gesäuert ist.

5. Verfahren nach einem der Ansprüche 1 bis 4, dadurch gekennzeichnet, dass das Kochen (10) 10 Minuten bis 1 Stunde dauert.

6. Verfahren nach einem der Ansprüche 1 bis 5, dadurch gekennzeichnet, dass das organische Lösemittel Aceton oder Dichlormethan ist.

7. Verfahren nach einem der Ansprüche 1 bis 6, dadurch gekennzeichnet, dass man eine Konzentrationsstufe (16) der im organischen Lösemittel enthaltenen Karotenoiden durchführt.

8. Extraktionsverfahren von Astaxanthin und seiner Esterderivate aus einer Kultur von *Haemotococcus pluvialis,* dadurch gekennzeichnet, dass man das Verfahren nach einem der Ansprüche 1 bis 7 verwendet.

## Claims

1. Process for extracting carotenoids from a culture of micro-algae, consisting in:
a) - centrifuging (2) the culture medium so as to form a paste rich in micro-algae;
b) - diluting (4, 6) the paste in a saline solution in order to cause an osmotic shock on the cells of the micro-algae;
c) - acidifying (8) and boiling (10) the solution in order to soften the cell wall;
d) - filtering (12) the solution in order to separate the acidified cells from the solution;
e) - washing (14) the cells obtained in d) by means of an organic solvent capable of solubilizing the carotenoids contained in the cells.

2. Process according to Claim 1, characterized in that the saline solution is a solution of NaCl.

3. Process according to Claim 2, characterized in that the saline solution contains from 50 to 300 g/l of NaCl.

4. Process according to any one of Claims 1 to 3, characterized in that the solution is acidified at a pH from 2 to 4.

5. Process according to any one of Claims 1 to 4, characterized in that boiling (10) is carried out for between 10 minutes and 1 hour.

6. Process according to any one of Claims 1 to 5, characterized in that the organic solvent is of acetone or dichloromethane.

7. Process according to any one of Claims 1 to 6, characterized in that a concentration stage (16) of the carotenoids contained in the organic solvent is carried out.

8. Process for extracting astaxanthine and its derived esters from a *Haemotococcus pluvialis* culture, characterized in that the process according to any one of Claims 1 to 7 is used.
